# EUROPÄISCHE PATENTSCHRIFT

(11) **EP 0 765 740 B1**
(45) Veröffentlichungstag und Bekanntmachung des Hinweises auf die Patenterteilung: **16.04.2003**
(21) Anmeldenummer: 96114437.5
(22) Anmeldetag: 10.09.1996
(51) Int. Cl.: A61L 29/00, B32B 7/02

(54) **PVC-freier-Mehrschichtschlauch für medizinische Zwecke, Verfahren zu dessen Herstellung und Verwendung**
PVC-free multilayered tube for medical uses, method for its manufacture and use
Tuyaux souples multicouches à usage médical, procédé pour leur fabrication et utilisation

(30) Priorität: 16.09.1995 DE 19534455
(43) Veröffentlichungstag der Anmeldung: 02.04.1997
(73) Patentinhaber: Fresenius AG, 61352 Bad Homburg v.d.H. (DE)
(72) Erfinder: Heilmann, Klaus, 66606 St. Wendel (DE); Nicola, Thomas, 57350 Spicheren (FR)
(74) Vertreter: Luderschmidt, Schüler & Partner GbR

(56) Entgegenhaltungen:
- EP-A- 0 533 493
- EP-A- 0 564 206
- EP-A- 0 738 589
- EP-A- 0 739 713
- US-A- 4 929 479
- US-A- 5 520 975
- SAECHTLING H.: "INTERNATIONAL PLASTICS HANDBOOK FOR THE TECHNOLOGIST, ENGINEER AND USER" 1992 , MUNICH, CARL HANSER VERLAG.; DE XP002033625 * Seite 177 *

## Beschreibung

Die Erfindung bezieht sich auf einen PVC-freien Mehrschichtschlauch für medizinische Zwecke gemäß Oberbegriff des Anspruchs 1, ein Verfahren zur Herstellung solcher PVC-freien Mehrschichtschläuche gemäß Anspruch 16 sowie die Verwendung der erfindungsgemäßen PVC-freien Mehrschichtschläuche.

Zum Stand der Technik werden die
- WO-A-92/11820: (= D1),
- DE-A-28 31 034: (= D2),
- US-A-4,948,643: (= D3),
- EP-A-0 136 848: (= D4),
- DE-PS-44 04 041: (= D5),
- DE-OS-42 19 071: (= D6),
- DE-OS-39 14 998: (= D7),
- WO-A-93/23093: (= D8),
- EP-A-0 564 206: (= D9),
- EP-A-0 533 493: (=D10),
- US-A-5,520,975: (=D11) und
- US-A-4,929,479: (=D12).
genannt.

PVC-freie-Materialien= Non-PVC-Materialien und daraus hergestellte einlagige Schläuche mit nur einer Schicht sind beispielsweise aus D1 bekannt. Hierin wird ein Schlauchmaterial für medizinische Zwecke vorgestellt, welches ein Blend aus Polyurethan und Polyester aufweist und das sowohl einer Sterilisationsbehandlung im Autoklaven unterzogen werden kann, heißsiegelfähig als auch mit hochfrequenter Energie versiegel- und verschmelzbar ist. Das beschriebene Schlauchmaterial ist dabei frei vom PVC-Weichmacher DEHP, einem Phthalat, das in den Verdacht geraten ist, krebserregende Eigenschaften zu besitzen. Es enthält jedoch gegebenenfalls als weichmachende Substanz bestimmte zusätzliche Anteile eines
Zitronensäureesters (Butyryltrihexylcitrat) und weitere Verarbeitungshilfsmittel wie innere oder äußere Gleitmittel. Das in D1 offenbarte thermoplastische Kunststoffmaterial kann zwar nach bekannten Formgebungsverfahren wie Extrusion, Spritzgießen oder Blasformen verarbeitet werden, ist im wesentlichen jedoch zur Verwendung mit medizinischen Beuteln oder Konnektoren aus PVC gedacht. Nur in Verbindung mit diesen "klassischen" PVC-Materialien zeigt es befriedigende Kompatibilität, insbesondere bei der Verbindungsbildung durch Heiß- oder Hochfrequenzversiegelung.

Auch aus der D2 sind von PVC- freie Kunststoffmassen bekannt, die zur Herstellung von Schläuchen zur Aufnahme oder Leitung von Blut oder medizinischen Lösungen geeignet sind. Gegenstand der D2 sind insbesondere Kunststoffmassen, die aus 10 bis 40 Gew.-% eines im wesentlichen aus Propyleneinheiten gebildeten Polyolefins, 40 bis 85 Gew.-% eines Blockcopolymeren aus einem zentralen Polyethylen oder Polybutylenblock mit endständigen Polystyrolblöcken, 10 bis 40 Gew.-% eines polymeren Weichmachers auf Polyethylenbasis und gegebenenfalls einem Antioxidationsmittel bestehen. Die offenbarten Materialien sind zwar flexibel, durchaus hitzebeständig, besitzen die für medizinische Anwendungszwecke geforderte Weichheit des Materials und können auch das im wesentlichen durch niedermolekulare Weichmacher verursachte Alterungsproblem größtenteils lösen, die Stabilität und Steifigkeit des Materials läßt jedoch zu wünschen übrig. Insbesondere ginge beim gezeigten Einschichtmaterial eine Erhöhung der Steifigkeit durch Anhebung des Polypropylenanteils zu Lasten der Weichheit und Flexibilität eines fertigen Schlauches oder Beutels.

Mehrschichtige Schläuche für medizinische Verbindungsleitungen sind Gegenstand der D3. Gezeigt werden dreischichtige Schläuche, deren äußere Schicht auf Ethylenvinylacetat (EVA) und deren innere Schicht auf Polyvinylchlorid (PVC) basiert. Da die Haftung zwischen innerer und äußerer Schicht nur mangelhaft ist, wird eine Haftvermittlungsschicht aus einem auf Ethylen basierenden Polymer, welches Vinylacetate und Acrylate enthält, als mittlere Schicht zusammen mit den beiden anderen Materialien coextrudiert. Kunststoffmaterialien mit der gezeigten Abfolge der Schichten eignen sich insbesondere zur Verwendung als Anschluß- oder Verbindungsstück oder -schlauch mit medizinischen Beuteln aus EVA (Kompatibilität der äußeren Schicht zum Beutel) und gestatten die Einführung und sichere Verbindung, beispielsweise durch Lösungsmittelverschmelzung, eines PVC- Membran-Schlauches innerhalb des Anschlußstückes oder -rohres. Bei den in D3 gezeigten Mehrschichtschläuchen handelt es sich aus medizinischer Sicht durchweg um bedenkliche Materialien, da die PVC- Schicht als Weichmacher erhebliche Mengen von Trimellithsäureestern aufweist, diese Verbindungen jedoch krebserzeugend sein können.

Aus D4 kennt man schließlich mehrschichtige Schläuche, die als potentieller Ersatz für PVC-Schläuche auf dem medizinischen Sektor angesehen werden. Allerdings wird die Verwendung von PVC gemäß der D4 nicht gänzlich ausgeschlossen, vielmehr wird es als Material oder Beimengung für eine Zwischenschicht oder die Innenschicht durchaus geduldet.

Im einzelnen betrifft die D4 einen dreischichtigen Schlauch für medizinische Zwecke, dessen innere Schicht aus Ethylen-Propylen-Copolymer, einem Polypropylen, einem Copolyester aus einem Polyether und einem Polyethylenterephtalat, Polyurethan, Polyvinylchlorid oder einem Blend aus Copolyester und Ethylen-Vinylacetat-Copolymer besteht.

Die Zwischenschicht kann aus einem LLDPE (Linear low density Polyethylene), Ethylen-Vinylacetat-Copolymer (EVAC), modifiziertem EVAC, Ethylen-Methylacrylat-Copolymer (EMAC), modifiziertem EMAC, PVC oder einem Blend der vorgenannten Verbindungen bestehen.

Die Außenschicht gemäß der D4 wird aus Polypropylen, Ethylen-Propylen-Copolymer oder modifiziertem Ethylen-Propylen-Copolymer gebildet.

Die Auswahl der Materialien für die Zwischenschicht richtet sich im wesentlichen nach der Fähigkeit dieser Materialien dem gesamten Aufbau des Mehrschichtschlauchs die notwendige Flexibilität zu verleihen. Das Kriterium für die Auswahl des Innenschichtmaterials ist eine ausreichende Hitzefestigkeit, um den resultierenden Schlauch autoklavierbar zu machen, während sich die Wahl eines Außenschichtmaterials im wesentlichen am Wunsch orientiert, eine relativ beständige Ultraschall-, Heiß- oder Hochfrequenzenergie-Versiegelung mit einem Polycarbonatkonnektor zu ermöglichen.

Abgesehen davon, daß die Schläuche gemäß D4 die Verwendung von PVC nicht gänzlich ausschließen, sind die Schläuche zwar mit Polycarbonat verträglich, jedoch weniger zur Verbindungsbildung mit anderen neueren und vorteilhafteren auf Polypropylen basierenden Beutel- oder Konnektormaterialien geeignet.

Darüber hinaus ist die flexible Mittelschicht gemäß der D4 üblicherweise die stärkste Schicht, was leicht eine zu geringe Steifigkeit des gesamten Schlauches mit sich bringt. So ist es in aller Regel und nicht nur wahlweise notwendig, die extrudierten Schläuche zu bestrahlen, um durch strahlungsinduzierte Vernetzung eine Hitzesterilisierbarkeit zu bewirken. Dieser Vorgang ist aufwendig.

Die D5 betrift Polymermaterial von medizinischen Instrumenten. Offenbart werden silangepropfte VLDPE's oder ULPDE's die mit Feuchtigkeit vernetzt wurden, um dann z.B. transparente und knickstabile sowie sterilisierbare Schläuche zu erhalten, insbesondere durch Extrusion. Hohe Vernetzungsgrade sind dabei eine Voraussetzung für die Dampfsterilisierbarkeit des Endprodukts. Obwohl das Material zum Ersatz von PVC geeignet scheint, fehlt es daraus erhältlichen Einschicht-Schläuchen an der Fähigkeit, mit einem Einbauteil eine gute und direkte Verbindung während einer einfachen Hitzesterilisationsbehandlung eingehen zu können, ohne die Formstabilität aufgeben zu müssen.

Die D6 offenbart strahlensterilisierbare recyclierbare Infusions- und Transfüsionsgeräte, bei denen sämtliche Bestandteile aus thermoplastischen oder elastomeren Homopolymeren, Copolymeren, Blockcopolymeren auf Basis von Polyolefinen dargestellt sind. Insbesondere macht die D6 der Öffentlichkeit auch Verbindungsschläuche aus PE-LLD oder aus linearem PE-LVLD zugängig, schließt aber die Verwendung von EVA oder speziellen Ionomeren nicht aus.

Zur Verbindung werden diese Schläuche unter Verwendung von organischen Lösungsmitteln wie Cyclohexan gefügt. Alternativ dazu lassen sich die Schläuche Ultraschallverschweißen oder mittels Licht- oder UV-härtbaren Klebern verbinden.

D7 befaßt sich mit Übertragungssystemen für Infüsions- oder Transfusionszwecke, bei denen zur umweltverträglichen Recyclierung sämtliche Bestandteile aus einem Polymeren, Copolymeren oder Blockcopolymeren unter Verzicht auf PVC bestehen. Die Polymeren Materialien, die Einsatz finden, basieren auf Styrol-Polymerisaten.

D8 betrifft PVC-freie coextrudierte Mehrschicht-Schläuche für medizinische Zwecke, die eine Kernschicht aus einem Blend aus Polyamid und EVA aufweisen. Auf dieser inneren Kernschicht ist eine äußere Schicht mittels einer Haftschicht aufgebracht. Die Haftschicht weist essentiell Copolyester und SEBS-Copolymer auf, optionell PP und EVA. Die Materialwahl belegt, daß der offenbarte Schlauch die Nachteile aufweisen muß, welche aus der Verwendung von EVA, Copolyester oder Polyamid resultieren.

Die D9 betrifft medizinische Behältnisse mit einem ein- oder mehrschichtigen Aufbau. Offenbart sind Strukturen, die eine Innen- sowie eine Außenschicht aufweisen, zwischen denen eine weitere Zwischenschicht sein muß. Alle Schichten enthalten kristalline Polyolefine, während die Zwischenschicht zusätzlich ein amorphes Polypropylen aufweisen muß. Außerdem offenbart die D9 Strukturen, die aus einer Schicht bestehen, welche wenigstens ein kristallines Polyolefin und amorphes Polypropylen aufweist.

Kristalline Polyolefine gemäß D9 sind iso- oder syndiotaktische Polypropylene oder ein Copolymer basierend darauf, isotaktisches Polybuten-1 oder ein isotaktisches Copolymer basierend darauf.

Amorphes Polypropylen ist ein ataktisches Polypropylen oder ein darauf basierendes Copolymer.

Die Zwischenschicht (bei 3 Schichten) oder die eine Schicht der einschichtigen Struktur können neben der Mischung aus kristallinem und amorphem Polyolefin zusätzlich eine Menge eines thermoplastischen Elastomers aufweisen.

Aus der D10 sind laminierte Folien bekannt, die eine Schicht (A) aufweisen, welche eine Harzzusammensetzung enthält, die 20 bis 100 Gew.-% amoprhes Polyolefin und 80 bis 0 Gew.-% kristallines Polypropylen aufweist; und die eine äußerste Schicht (B) aufweist, die ein kristallines Polypropylen, ein Ethlyen(co)polymer oder ein kristallines Polypropylen und ein polares Ethylen(co)polymer enthält.

D11 offenbart Mehrschichtfolien für medizinische Anwendungen mit innerer, äußerer und Zwischenschicht, wobei alle Schichten vorwiegend auf Polyolefinen beruhen und die innere Schicht aus einer Mischung aus Ethlyen-α-Olefin Copolymer mit ϕ = 0,930 bis 0,945 g/cm³ und einem Polypropylen besteht, die Mittelschicht aus einer Mischung aus einem Ethlyen-α-Olefin-Copolymer mit ϕ = 0,920 bis 0,945 g/m³ mit einem Ethlyen-α-Olefin Elastomer mit einer Dichte ϕ = 0,880 bis 0,890 g/cm³, während die äußere Schicht aus einem Ethylen-α-Olefincopolymer mit einer Dichte von 0,930 bis 0,945 g/cm³ gebildet wird.

D12 schließlich bezieht sich auf einen medizinischen Beutel, der aus einem Laminat besteht, welches eine innere Schicht aus einem Polyethylen niedriger Dichte, eine Zwischenschicht aus EVA, EP-Elastomer oder Ethlyen-Buten-1-Elastomer und eine Außenschicht aus PE niedriger Dichte umfaßt.

Angesichts der Nachteile, mit denen die im dargelegten Stand der Technik beschriebenen Ausführungsformen behaftet sind, ist es Aufgabe der vorliegenden Erfindung, einen Schlauch für medizinische Zwecke zur Verfügung zu stellen, der eine individuelle Anpassung an viele verschiedene, insbesondere auf Polypropylen oder Polycarbonat basierenden Konnektor- oder Beutelmaterialien gestattet. Ferner ist es wünschenswert, daß mit dem neuen Schlauchmaterial eine feste Bindung ohne zusätzlichen Haftvermittler oder dergleichen möglich ist. Zusätzlich soll der neue Mehrschichtschlauch ausreichend flexibel, elastisch und weich sein und trotzdem möglichst wenig knickanfallig, relativ formstarr und thermisch stabil sein. Schließlich soll der Schlauch bei Berührung mit den in der Medizin üblichen Fluiden auch keine gefährlichen Substanzen in diese Fluide abgeben und überhaupt inert gegenüber medizinischen Lösungen sein. Ziel der Erfindung ist es außerdem, ein Verfahren zur Herstellung eines solchen Mehrschichtschlauches anzugeben. Weiterhin ist die Angabe von Verwendungsmöglichkeiten des erfindungsgemäßen Schlauchmaterials Aufgabe der Erfindung.

Diese und weitere nicht näher genannte Aufgaben werden durch einen PVC-freien Mehrschichtschlauch für medizinische Zwecke der eingangs erwähnten Art mit den Merkmalen des kennzeichnenden Teils des Anspruchs 1 gelöst. Besondere Weiterentwicklungen werden in den von Anspruch 1 abhängigen Ansprüchen unter Schutz gestellt. In verfahrensmäßiger Hinsicht wird die erfindungsgemäße Aufgabe durch ein Verfahren gemäß Anspruch 16 gelöst, während die Ansprüche 19 bis 21 die Verwendung des erfindungsgemäßen PVC-freien Mehrschichtschlauches schützen.

Dadurch, daß bei einem PVC-freien Mehrschichtschlauch für medizinische Zwecke mit wenigstens zwei Schichten, von denen eine Basisschicht A) aus einem ersten Kunststoffmaterial mit wenigstens einer Konnektionsschicht B) aus einem zweiten Kunststoffmaterial verbunden ist, das erste Kunststoffmaterial zum überwiegenden Teil ein synthetischer Kautschuk auf Isopren-Basis oder Polypropylen mit einer Dichte ρ ≤ 0,9 g/cm³, welches eine Hitzesterilisation bei ≥ 121°C ohne Formschädigung übersteht, eine Härte Shore D ≤ 32 hat, das bei ≥ 121°C eine
Restspannung aufweist, die ausreicht, um an einer Konnektionsstelle einen Preßssitz zu bilden, so daß die Basisschicht A) bei Temperaturen ≥ 121°C noch formbeständig ist, und aus dem ein Kringel oder eine Schlaufe mit einem Durchmesser von bis zu 60 mm ohne Knickschädigung geformt werden kann, während das zweite Kunststoffmaterial zum überwiegenden Teil ein Polyethylen Copolymer,
oder ein synthetischer Kautschuk mit M_{w} < 100 000 g/mol ist, welches während einer Hitzesterilisation bei 121°C unter dem bei Ausbildung eines Preßsitzes resultierenden Konnektionsdruck zum Fließen neigt und eine Härte Shore A ≤ 65 aufweist, so daß jede Konnektionsschicht B) bei einer Temperatur von 121°C unter Konnektionsdruck nicht formbeständig ist, gelingt es einem flexiblen Schlauch, der nach erfolgter Hitzesterilisation transparent ist, der ausreichende Knickstabilität aufweist und der mit Schlauchklemmen oder dergleichen abdichtbar ist, zur Verfügung zu stellen. Darüber hinaus hat der erfindungsgemäße PVC-freie Mehrschichtschlauch zusätzlich die Fähigkeit mit einem Einbauteil, einem medizinischen Beutel oder einem Konnektor eine feste und dichte Verbindung einzugehen, und zwar äußerst einfach während einer möglichen Hitzesterilisationsbehandlung.

Der vorliegenden Erfindung liegt mithin unter anderem der Gedanke zugrunde, verschiedene Kunststoffschichten in einem mehrschichtigen Schlauchmaterial so aufeinander abzustimmen, daß wenigstens eine Schicht als Basisschicht fungierend dem Schlauchmaterial eine genügende thermische Stabilität bei der Sterilisation verleiht, während wenigstens eine andere Schicht als Konnektions- oder Verbindungsschicht fungierend, die Ausbildung einer festen und dichten Verbindung mit einem Beutel, Anschlußstutzen, Konnektor oder einem anderen Schlauch ermöglicht, ohne daß zusätzliche Haft-, Dichtungs- oder Versiegelungsmassen oder Hilfsmittel einzusetzen wären oder andere Versiegelungsverfahren (hochfrequente Energie oder ähnliches) anzuwenden wären.

Im Rahmen der Erfindung wird dabei unter "Sterilisation" allgemein ein Verfahren für die Abtötung bzw. Inaktivierung (Viren) aller Mikroorganismen einschließlich der hochresistenten Dauerformen verstanden, wobei die erfindungsgemäßen Schläuche insbesondere einer Dampf-Sterilisation im Autoklaven mit gespanntem Wasserdampf von mindestens 121°C, entsprechend etwa einer Atmosphäre Überdruck, der sogenannten Autoklavierung oder Autoklavierungsbehandlung ohne Schaden zu nehmen widerstehen.

Weiterhin werden für die Erfindung unter "Kunststoffmaterial" solche Materialien verstanden, deren wesentliche Bestandteile aus makromolekularen organischen Verbindungen bestehen, wobei die Kunststoffmaterialien auch als Polymere bezeichnet werden, wozu insbesondere Homopolymere als auch Copolymere (statistische, Block- und/oder Pfropfpolymere) sowie Mischungen (= Blends) aus den vorgenannten Stoffen gehören.

Ein wichtiges Kriterium für die erfindungsgemäße Auswahl und Zuordnung eines Polymers zu einem Kunststoffmaterial und damit zu einer bestimmten Funktionsschicht des mehrschichtigen PVC-freien Schlauchs für medizinische Zwecke stellt die Formbeständigkeit während der Hitzesterilisation dar.

Ein Kunststoffmaterial gilt in diesem Sinne als formbeständig, wenn eine Schlauchprobe von mindestens 10 mm Länge, einem Innendurchmesser von 5 mm und einem Außendurchmesser von 7 mm eine Heißdampfsterilisation bei 121°C mit mindestens 15 min. Aufheizzeit, mindestens 15 min. Haltezeit und mindestens 10 min. Kühlzeit ohne optisch sichtbare Formveränderung wie "Zusammenfallen" oder "Ovalität" etc., übersteht.

Die Temperatur, die für die Erweichung der erfindungsgemäßen Polymere oder Kunststoffmaterialien von Interesse ist, ist die Temperatur der Dampfsterilisation, nämlich 121°C. Dadurch, daß die Basisschicht ein Polymer aufweist, dessen Formbeständigkeit also Temperaturbeständigkeit bezüglich Deformation auch bei mehr als 121°C gegeben ist, wird die Möglichkeit des fließfähigen Erweichens oder des Erreichens des flüssigen Zustandes der Basisschicht bei der Dampfsterilisation weitestgehend ausgeschlossen, während ein Polymer des Kunststoffmaterials der Konnektionsschicht dessen fließfähiges Erweichen unter Preßdruck bei 121°C erreicht wird das Erweichen der Konnektionsschicht unter den Standardbedingungen der Dampfsterilisation zuläßt. Dadurch wird an einer Kontaktstelle mit einem Verbindungspartner eine Verbindung mit diesem möglich, ohne daß die Form des Schlauches sich unkontrolliert ändert.

Die angegebenen Temperaturen beziehen sich dabei jeweils auf den Druck bei der Dampfsterilisation, also circa eine Atmosphäre Überdruck. Es versteht sich jedoch, daß die Druckabhängigkeit der Erweichungstemperatur im betrachteten Bereich zwischen Normaldruck und dem zum Dampfsterilisieren nötigen Überdruck im allgemeinen vernachlässigbar gering ist.

Je nach gewünschter Funktion des erfindungsgemäßen PVC-freien Schlauchs, kann es von Vorteil sein, die speziell zu Verbindungsbildung befähigte Schicht B) außen, innen oder sowohl außen als auch innen anzuordnen. Demnach ist der PVC-freie Mehrschichtschlauch der Erfindung bevorzugt durch die Abfolge der Schichten B) A), A) B) oder B) A) B), jeweils von innen nach außen, gekennzeichnet.

Im ersten Fall kann der erfindungsgemäße Schlauch z. B. auf einen Konnektor aus geeignetem Material "aufgesteckt" werden, so daß die Innenschicht des Schlauchs mit der Außenoberfläche des Konnektors in Berührung ist. Im zweiten Fall ist der erfindungsgemäße Schlauch zum Einführen in ein Hohlteil gedacht, dessen Innenoberfläche aus geeignetem Material zur Ausbildung einer Verbindung besteht, während bei Anordnung zweier Verbindungsschichten (außen und innen) beim erfindungsgemäßen PVC-freien Mehrschichtschlauch die beiden genannten Verbindungsmöglichkeiten wahlweise oder gleichzeitig realisierbar sind. Bevorzugt ist bei einer Verbindung mit Konnektor die Kennektionsschicht innen, während bei einer Verbindung z.B. mit einem Beutel die Konnektionsschicht außen ist.

Neben den bislang beschriebenen Basis- und Konnektionsschichten verfügt der PVC-freie Mehrschichtschlauch der Erfindung in einer bevorzugten Weiterbildung über eine weitere Funktionsschicht, nämlich über zusätzlich wenigstens eine transparente Deckschicht C) als Außenschicht aus einem dritten Kunststoffmaterial, wobei eine Schicht C) als innerste oder äußerste Schicht angeordnet sein kann.

Die Deckschicht verleiht dem Mehrschichtschlauch eine verbesserte Oberflächenbeschaffenheit hinsichtlich Klebrigkeit, Stumpfheit und verringertem Reibwert, Transparenz und spezielle Siegeleigenschaft.

Grundsätzlich kann die Schicht C) als Abschlußschicht entweder innen oder außen vorgesehen sein. Es ist für die Erfindung jedoch bevorzugt, daß für den Fall, daß die äußerste oder innerste Außenschicht eine Konnektionsschicht B) ist, die dazu korrespondierende entgegengesetzt angeordnete Außenschicht eine Schicht C) ist.

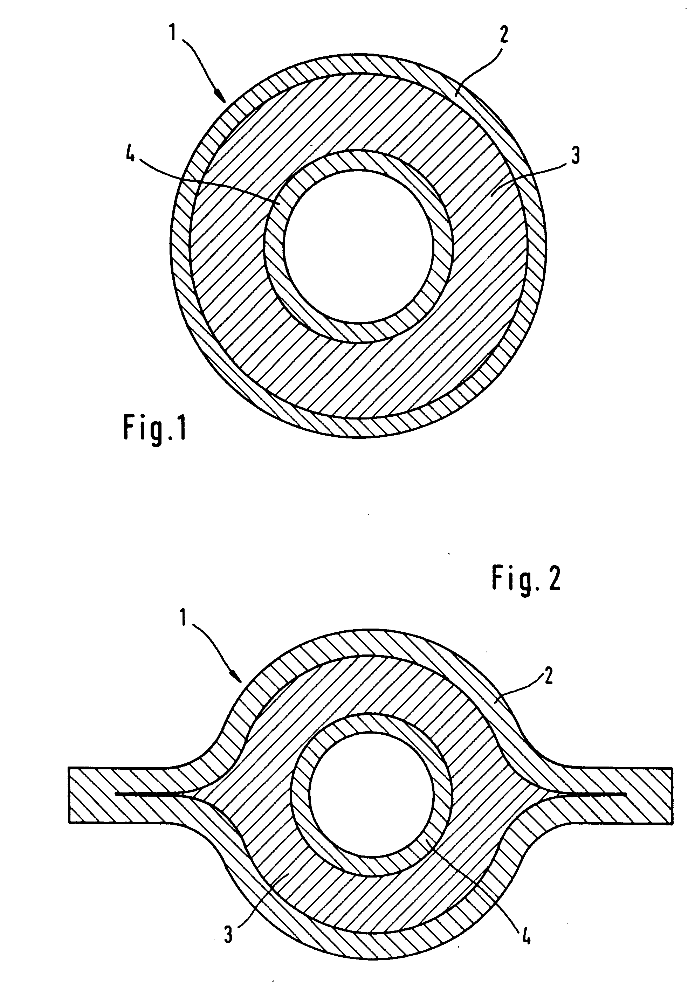

Besonders vorteilhaft ist zumindest an der Deckschicht eine Ausbildung als Lippenschlauch (Fig. 2). Mithin sind erfindungsgemäß besonders bevorzugte Ausführungsformen solche PVC-freien Mehrschichtschläuche, die durch die Abfolge der Schichten B) A) C)/ C) A) B)/ C) B) A) B) oder B) A) B) C), jeweils von innen nach außen, gekennzeichnet sind. Je nach Verwendung des Schlauches muß die innere Schicht mit der durch den Schlauch fließenden Lösung kompatibel sein.

Um die vorerwähnten Eigenschaften der Schicht C) zu gewährleisten, besteht in weiter vorteilhafter Ausführungsform die Deckschicht C) aus einem dritten Kunststoffmaterial, das wenigstens ein Polymer aufweist, dessen Temperaturbeständigkeit gegenüber Deformierung noch über > 121°C gegeben ist.

Bei erfindungsgemäß bevorzugten PVC-freien Mehrschichtschläuchen ist das erste Kunststoffmaterial für die Basisschicht zum überwiegenden Teil ein synthetischer Kautschuk auf Isopren-Basis oder Polypropylen mit einer Dichte ρ ≤ 0,9 g/cm³ und das zweite Kunststoffmaterial für die Konnektionsschicht zum überwiegenden Teil ein Polyethylen Copolymer oder ein synthetischer Kautschuk mit Mw < 100 000 g/mol. Diese Kombination für jede Basisschicht A) und die Konnektionsschicht oder -schichten B) kann bereits eine Vielzahl an geforderten Eigenschaften bieten. Für die Erfindung besonders vorteilhaft ist eine Polymerverwendung gemäß der folgenden Auflistung. Prozentangaben bedeuten jeweils Gewichtsprozent.
- Deckschicht:: Dicke = 10 - 50µm
- 40% - 60% PP-R: (PP23M 10 cs 264, REXENE) und
- 60% - 40% SIS: (HVS 3, Kuraray); oder Tuftec H 1052 (Asahi)

- Basisschicht:: Dicke : 900 - 980 µm bei ca. 1 mm Wandstärke des Schlauchs

- 50 - 100% SIS: (HVS/3, Kuraray) und
- 50 - 0% PP-R: (PP 23M10cs264, REXENE); - PP mit Shore D ≤ 32, Q = 0,9 g/cm³; (z.B. Adflex 100 G,
Himont, mit bis zu 50% Kautschukanteil, z.B. PIB, Styrol-Ethylen-Butylen-Kautschuk, Styrol-Ethylen-Propylen-Kautschuk, SIS)
- Konnektionsschicht:: Dicke 10 - 50 µm

- 100% SEBS-Compound: (PR 3415, Wittenburg)
- 100 % SEPS/SEP: (Septon 2277, Kuraray) oder
- 50 - 100% PE-Copolymer: (Engage XU58.000 52, DOW) und
- 0 - 50% SEBS/SEB: (Kraton G 1726, Shell);

Die Abkürzungen bedeuten im einzelnen:
- PP-R =: Polypropylen-Randomcopolymer
- SIS =: Styrol-Isopren-Styrol
- SEBS =: Styrol-Ethylen-Butylen-Styrol-Kautschuk
- SEB =: Styrol-Ethylen-Butylen-Kautschuk
- SEPS =: Styrol-Ethylen-Propylen-Styrol-Kautschuk
- SEP =: Styrol-Ethylen-Propylen-Kautschuk
- PE-Copolymer =: Polyethylen-Copolymer

SEBS und SEB gehören zur Gruppe der Styrol-Ethylen-Butylen-Blockcopolymere, welche im Rahmen der Erfindung mit Vorteil als Konnektionsschichtmaterialien oder in der Konnektionsschicht in Mischung mit anderen Materialien jeweils auch in unterschiedlichen Blockanteilen eingesetzt werden können.

SEBS-Compound is ein Gemisch verschiedener Styrol-Ethylen-Butylen-Blockcopolymere.

Gleiches gilt für SEPS und SEP, welche zur Gruppe der Styrol-Ethylen-Propylen-Blockcopolymere gehören. Ebenso ist ein SEPS-Compound ein Gemisch verschiedener Styrol-Ethylen-Propylen-Blockcopolymere.

Wiederum kann im Hinblick auf die Haftung zwischen Schichten aus den Materialien A), B) und C) gesagt werden, daß diese grundsätzlich ausreichend ist. Sie kann jedoch in vorteilhafter Weise dadurch gesteigert werden, daß die Schichten A), B) und/oder C) jeweils zusätzlich bis zu 40 Gew.-%, bezogen auf 100 Gew.-% ihrer wie oben beschriebenen und definierten Zusammensetzung, desjenigen Kunststoffmaterials aufweisen, welches zur Ausbildung einer oder beider benachbarten Schichten des PVC-freien Mehrschichtschlauches dient. Vorteilhaft ist auch eine weitere Zwischenschicht aus den Polymermaterialien der jeweils benachbarten Schichten.

Durch diese "Materialvermittlung" bzw. die Substitution von Material wird die Kompatibilität der miteinander zu einem Schlauch geformten Schichten deutlich gesteigert, ohne die sonstigen Eigenschaften in Frage zu stellen.

Ein weiteres wesentliches und besonders bevorzugtes Merkmal der Erfindung ist darin zu sehen, daß in Weiterbildung des erfindungsgemäßen PVC-freien Mehrschichtschlauchs die Kunststoffmaterialien für alle Schichten des Schlauchs so gewählt sind, daß sie im wesentlichen aus Polyolefinhomopolymeren oder Polyolefincopolymeren oder Abwandlungen davon (z.B. SEBS) bestehen. Es war insbesondere erstaunlich, daß es mit der Erfindung erstmals gelingt, einen PVC-freien Mehrschichtschlauch zu schaffen, der ausschließlich aus umweltverträglichen Materialien besteht, die problemlose Verbindungsbildung mit Konnektoren bei der Dampfsterilisation gestatten und gleichzeitig alle weiteren Forderungen an einen medizinisch einsetzbaren Schlauch erfüllen.

Hinsichtlich der Geometrie können die Schläuche selbst in allen erforderlichen und üblichen Dicken und Größen ausgeführt sein. Bevorzugt besteht der erfindungsgemäße PVC-freie Mehrschichtschlauch zu mehr als 96-98 Vol.-%, bezogen auf das gesamte Volumen des Schlauchmaterials aus der Basisschicht A). Die einzelnen Schichten selbst weisen bevorzugt folgende Dicken auf: Die Basisschicht A) eine Dicke > 900 µm, die Konnektionsschicht B) eine Dicke zwischen 10 µm und 50 µm und die Siegelschicht C) eine Dicke zwischen 10 µm und 50 µm.

Gegenstand der Erfindung ist auch ein Verfahren zur Herstellung eines PVC-freien Mehrschichtschlauches für medizinische Zwecke, bei dem man zur Erzeugung einer wenigstens zweischichtigen Mehrschichtfolie aus Kunststoff ein erstes Kunststoffmaterial zur Ausbildung einer Basisschicht A) und ein zweites Kunststoffmaterial zur Ausbildung wenigstens einer damit verbundenen Konnektionsschicht B) miteinander koextrudiert und einen im wesentlichen koaxialen und zylindrischen Mehrschichtschlauch formt, wobei das Verfahren dadurch gekennzeichnet ist, daß man ein erstes Kunststoffmaterial verwendet, das wenigstens ein Polymer aufweist, das eine Hitzesterilisation bei ≥ 121°C ohne Formschädigung übersteht, eine Härte Shore D ≤ 32 hat, das bei ≥ 121°C eine Restspannung aufweist, die ausreicht, um an einer Konnektionsstelle einen Preßsitz zu bilden, und aus dem eine Kringel oder eine Schlaufe mit einem Durchmesser von bis zu 60 mm ohne Knickschädigung geformt werden kann, während man ein zweites Kunststoffmaterial verwendet, das wenigstens ein Polymer aufweist, das während einer Hitzesterilisation bei 121°C unter dem bei Ausbildung eines Preßsitzes resultierenden Konnektionsdruck zum Fließen neigt und eine Härte Shore A ≤ 65 aufweist, so daß die Formbeständigkeit des ersten Kunststoffmaterials bei Temperaturen ≥ 121°C gegeben ist und die des zweiten Kunststoffmaterials nicht mehr.

Die Formgebung erfolgt dabei nach dem Fachmann geläufigen Verfahren, wie beispielsweise der Vakuumkalibrierung. Wichtig für die Erfindung ist vor allem, daß es durch die Koextrusion mehrerer Schichten miteinander gelingt, mehrere gewünschte Eigenschaften von Einzelkomponenten in einem Schlauch zu vereinigen, um dabei die Qualität des Schlauches zu steigern.

Hierfür bietet gerade das Koextrusionsverfahren die Möglichkeit unter geeigneter Auswahl der Extrusionspartner maßgeschneiderte PVC-freie Mehrschichtschläuche zur Verfügung zu stellen, die es auf einzigartige Weise gestatten, auf jeglichen Zusatz von Haftvermittlern zu verzichten und dennoch die geforderten Eigenschaften und zusätzlich darüber hinaus andere wichtige Eigenschaften wie Gas- und Wasserdampfdurchlässigkeit, Festigkeit des Materials, Schweißbarkeit, Transparenz und Temperaturbeständigkeit des Schlauches zu beeinflussen.

Von großer Bedeutung für die Erfindung ist die Auswahl der miteinander zu extrudierenden Schichtmaterialien, wobei besonders bevorzugt Kunststoffmaterialien oder Schichten so ausgewählt werden, daß alle Schichten des PVC-freien Mehrschichtschlauchs im wesentlichen aus Polyolefinhomopolymeren und/oder Polyolefincopolymeren bestehen, oder aus darauf basierenden Polymeren wie z.B. Abwandlungen von Polyolefinen (z.B. SEBS).

Die Koextrusion solcher Materialien ist zwar grundsätzlich bekannt, es ist jedoch aufgrund vorhandener Erfahrungen nicht ohne weiteres die erfolgreiche Realisierung eines so komplexen Mehrschichtschlauches, wie des erfindungsgemäßen, vorhersehbar gewesen. Insofern war der erfindungsgemäße Erfolg überraschend, da sich in der Praxis sonst immer wieder zeigte, daß selbst bei Zuhilfenahme von gegebenenfalls tabellierten Eigenschaften von Polymeren, etwa Daten zur Verbundhaftung, der Einsatz solcher Materialien nicht notwendigerweise zum Erfolg führt. Das heißt, die Lösung einer gestellten Aufgabe durch reine Auswahl aus bekannten Materialien ist bei einem mehrschichtigen Koextrusionsschlauch grundsätzlich nicht ohne weiteres möglich. So ist die Abstimmung der Viskosität der Schmelze bei der Coextrusion von Kautschuken wie PIB mit PP besonders schwierig.

Weiterhin ist es beim erfindungsgemäßen Verfahren bevorzugt, daß man zur Formung eines PVC-freien Mehrschichtschlauchs die Kunststoffmaterialien so auswählt, daß alle Schichten des Schlauches zusätzlich bis zu 40 Gew.-% des Materials der jeweils benachbarten Schicht oder Schichten enthalten können. Hierdurch kann in gewissem Maße eine geringe Haftung zwischen zwei benachbarten Schichten kompensiert werden. Nach der eigentlichen Formung kann der erhaltene Schlauch auf übliche Arten weiterbearbeitet werden. Bevorzugt wird er nach dem Formen mit Wasser schockgekühlt. Hierdurch wird wegen des "Einfrierens" des amorphen Zustandes ein optimaler Verbund mit hoher Flexibilität und ausreichender Steifigkeit erreicht, aber vor allem wird durch das schockartige Kühlen der Schmelze Transparenz des Schlauches verbessert, weil sich keine kristallinen Bereiche ausbilden können. Dies führt zu einem geringen Kristallinitätsgrad und damit zur hohen Transparenz und Zähigkeit.

Der erfindungsgemäße PVC-freie Mehrschichtschlauch eignet sich hervorragend für eine Verwendung im medizinischen Bereich. Die Materialien des Mehrschichtschlauchs sind alle so ausgewählt, daß der Schlauch durchsichtig, knickbeständig und flexibel, insbesondere aber heißsterilisierbar und durch gleichzeitig vom Schlauch ausgeübte Preßkraft mit einem entsprechenden Konnektor fest und bakteriensicher verbindbar ist. Zusätzlich ist der Non-PVC-Mehrschichtschlauch der Erfindung auch noch biokompatibel. Die Verwendung von PVC, welches immer einen Anteil an Weichmachern enthält, wird vermieden, ebenso werden keine Haftvermittler benötigt, die gegebenenfalls durch die Kunststoffmaterialschichten hindurchdiffundieren könnten.

Aufgrund seiner hervorragenden Material- und Gebrauchseigenschaften wird der erfindungsgemäße PVC-freie Mehrschichtschlauch mit besonders großem Vorteil als fluidführende Leitung in der Dialyse, Infusion oder künstlichen Ernährung verwendet. Zu diesem Zweck ist es vorteilhafterweise vorgesehen, daß zumindest der Anschlußbereich zum Anschluß an den Vorratsbeutel mit einer Schweißlippe versehen ist.

Hierbei macht man sich vor allem die Kompatibilität des Konnektionsschichtmaterials des erfindungsgemäßen Mehrschichtschlauchs mit den Anschlußstücken von medizinischen Beuteln (vor allem aus Polypropylen) und/oder speziell in der Medizintechnik üblichen Verbindungstechniken, beispielsweise in Form von Konnektoren aus Polypropylen zu nutzen. Konnektoren oder Beutel können dabei mit einer rauhen Oberfläche versehen sein, auf die ein erfindungsgemäßer PVC-freier Mehrschichtschlauch gesteckt wird, so daß die Innenoberfläche des PVC-freien Mehrschichtschlauches mit der Konnektionsschicht B) des Schlauches einen Preßsitz mit der rauhen Außenoberfläche des Konnektors oder Beutels (gegebenenfalls Anschlußstutzen des Beutels) bildet.

Durch die Oberfläche der Polypropylenteile sowie die Fließeigenschaft der Konnektionsschicht B) des Schlauches bei der Einwirkung von Hitze etwa bei der Dampfsterilisation ist durch Einfließen des Materials der Konnektionsschicht in die Unebenheiten der Oberfläche des Konnektors oder Beutelstutzens eine bereits gute und feste Verbindungsausbildung gegeben. Noch besser wird die Verbindung, wenn die zur Herstellung der Konnektionsschicht B) des Schlauches verwendeten Kunststoffmaterialien mit einem Anteil zwischen 1 und 40 Gew.-%, bezogen auf 100 Gew.-% des Materials der Konnektionsschicht mit einem Kunststoffmaterial, aus welchem der Konnektor oder der Anschlußstutzen des Beutels besteht, geblendet werden. Die Verbindung kann durch Aufrauhen der Oberfläche verbessert werden.

Weitere Vorteile und Einzelheiten der Erfindung ergeben sich aus den nachfolgenden Beispielen, die unter Bezugnahme auf die beigefügten Figuren erläutert werden.

In den Figuren zeigen
Figur 1 einen Querschnitt einer erfindungsgemäßen Ausführungsform des PVC-freien Mehrschichtschlauches,
Figur 2 eine weitere Ausführungsform mit Lippenbildung.

In den Figuren sieht man einen dreischichtigen Schlauch 1 gemäß der vorliegenden Erfindung. Die Außenschicht 2 des Schlauches 1 ist bei der in Figur 1 gezeigten Ausführungsform eine Deckschicht C) aus einem Blend aus SEBS-Compound, SEPS-Compound, ein PP/SIS-Blend oder PP/Styrol-Ethylen-Butylen- (-Propylen) Kautschuk-Blend, SEBS und/oder SEPS.

Die Zwischenschicht 3 stellt als Basisschicht A) das größte Volumen der Schlauchhülle dar. Geeignete Materialien sind unter anderem Styrol-Ethylen-Butylen-Kautschuk mit PP, PIP mit PP, SEPS mit PP, PP und SIS mit PP.

Die Konnektionsschicht 4 besteht aus SEBS-Compound mit SEBS/SEB (Kraton G 1726 und Kraton G 1652, Shell), einem PE-Copolymer (Engage XU58000, 52, DOW) gegebenenfalls mit SEBS/SEB (Kraton G 1726, Shell) oder SEPS/SEP (Septon 2277, Kuraray). Kraton G 1726 ist dabei ein niedermolekulares SEBS mit mindestens 20% Diblock-Anteil, während Kraton G 1652 ein höhermolekulares SEBS ohne nenneswerte Diblockanteile ist.

Die Schichten A), B) und C) weisen dabei folgende Formbeständigkeiten, E-Module, und Härten auf:

| | E-Modul | Härte | Formbeständigkeit bei Hitzesterilisation |
|---|---|---|---|
| Hauptschicht Basis A) | ≤ 80N/mm² | Shore D≤32 | >123°C |
| Konnektionsschicht B) | ≤ 80N/mm² | Shore A≤65 | <121°C |
| Deckschicht C) | <1000N/mm² | <R90*) | >123°C |

| | | | |
|---|---|---|---|
| *) R90 bedeutet Härte 90 nach Rockwell; vgl. DIN 10109-1 | | | |

Weitere Vorteile und Ausführungsformen ergeben sich aus den nachfolgenden Patentansprüchen.

## Patentansprüche

1. PVC-freier Mehrschichtschlauch für medizinische Zwecke mit wenigstens zwei Schichten, von denen eine Basisschicht A) aus einem ersten Kunststoffmaterial mit wenigstens einer Konnektionsschicht B) aus einem zweiten Kunststoffmaterial verbunden ist,
**dadurch gekennzeichnet, daß**
das erste Kunststoffinaterial zum überwiegenden Teil ein synthetischer Kautschuk auf Isopren-Basis oder Polypropylen mit einer Dichte p ≤ 0,9 g/cm³ ist, welches eine Hitzesterilisation bei ≥ 121 °C ohne Formschädigung übersteht, eine Härte Shore D < 32 hat, das bei ≥ 121°C eine Restspannung aufweist, die ausreicht, um an einer Konnektionsstelle einen Preßsitz zu bilden, so daß die Basisschicht A) bei Temperaturen ≥ 121°C noch formbeständig ist und aus dem ein Kringel oder eine Schlaufe mit einem Durchmesser von bis zu 60 mm ohne Knickschädigung geformt werden kann, während das zweite Kunststoffmaterial zum überwiegenden Teil ein Polyethylen Copolymer oder ein synthetischer Kautschuk mit M_{w} < 100.000 g/mol ist, welches während einer Hitzesterilisation bei 121°C unter dem bei Ausbildung eines Preßsitzes resultierenden Konnektionsdruck zum Fließen neigt und eine Härte Shore A ≤ 65 aufweist, so daß jede Konnektionsschicht B) bei einer Temperatur von 121°C unter Konnektionsdruck nicht mehr formbeständig ist.

2. PVC-freier Mehrschichtschlauch nach Anspruch 1, **gekennzeichnet durch** die Abfolge der Schichten B) A), A) B) oder B) A) B), jeweils von innen nach außen.

3. PVC-freier Mehrschichtschlauch nach einem der vorhergehenden Ansprüche, **dadurch gekennzeichnet, daß** der Schlauch zusätzlich wenigstens eine transparente Deckschicht C) als Außenschicht aus einem dritten Kunststoffmaterial aufweist, wobei die Schicht C) jeweils als innerste oder äußerste Schicht angeordnet ist.

4. PVC-freier Mehrschichtschlauch nach Anspruch 3, **dadurch gekennzeichnet, daß** die äußerste oder innerste Außenschicht eine Konnektionsschicht B) und die dazu korrespondierende entgegengesetzte Außenschicht eine Deckschicht C) ist.

5. PVC-freier Mehrschichtschlauch nach einem der Ansprüche 3 oder 4, **gekennzeichnet durch** die Abfolge der Schichten B) A) C), C) A) B), C) B) A) B) oder B) A) B) C) jeweils von innen nach außen.

6. PVC-freier Mehrschichtschlauch nach Anspruch 3 oder 4, **gekennzeichnet durch** die Schichten B)A)C) oder C)A)B) je nach Verwendung des Schlauches.

7. PVC-freier Mehrschichtschlauch nach einem der vorhergehenden Ansprüche 3 bis 6, **dadurch gekennzeichnet, daß** das dritte Kunststoffmaterial wenigstens ein Polymer aufweist, dessen Formbeständigkeit bei Temperaturen ≥ 121°C gegeben ist.

8. PVC-freier Mehrschichtschlauch nach Anspruch 7, **dadurch gekennzeichnet, daß** das dritte Kunststoffmaterial bei Temperaturen ≥ 121°C Formbeständigkeit aufweist.

9. PVC-freier Mehrschichtschlauch nach einem der vorhergehenden Ansprüche, **dadurch gekennzeichnet, daß** die Schichten ohne zusätzlichen Haftvermittler zusammenhaften.

10. PVC-freier Mehrschichtschlauch nach einem der vorhergehenden Ansprüche, **dadurch gekennzeichnet, daß** die Schichten im wesentlichen frei von Weichmachern, Antiblockmitteln, Antistatika sowie anderen Füllstoffen sind.

11. PVC-freier Mehrschichtschlauch nach einem der Ansprüche 3 bis 10, **dadurch gekennzeichnet, daß** die Schichten A), B) und/oder C) jeweils zusätzlich bis zu 40 Gew.-% bezogen auf 100 Gew.-% ihres Kunststoffmaterials, desjenigen Kunststoffmaterials aufweisen, welches zur Ausbildung einer oder beider benachbarten Schichten des PVC-freien Mehrschichtschlauches dient.

12. PVC-freier Mehrschichtschlauch nach einem der vorhergehenden Ansprüche, **dadurch gekennzeichnet, daß** die Kunststoffmaterialien für alle Schichten des Schlauches so gewählt sind, daß sie im wesentlichen aus Polyolefin oder aus darauf basierenden Kunststoffen bestehen.

13. PVC-freier Mehrschichtschlauch nach einem der vorhergehenden Ansprüche, **dadurch gekennzeichnet, daß** die Basisschicht A) eine Dicke zwischen 900 und 980 µm aufweist.

14. PVC-freier Mehrschichtschlauch nach einem der vorhergehenden Ansprüche, **dadurch gekennzeichnet, daß** die Konnektionsschicht B) eine Dicke zwischen 10 µm und 50 µm aufweist.

15. PVC-freier Mehrschichtschlauch nach einem der Ansprüche 4 bis 14, **dadurch gekennzeichnet, daß** die Deckschicht C) eine Dicke zwischen 10 µm und 50µm aufweist.

16. Verfahren zur Herstellung eines PVC-freien Mehrschichtschlauches für medizinische Zwecke gemäß den Ansprüchen 1-15, bei dem man zur Erzeugung eines wenigstens zweischichtigen Mehrschichtschlauchs aus Kunsstoff das erste Kunststoffmaterial zur Ausbildung der Basisschicht A) und das zweite Kunststoffmaterial zur Ausbildung wenigstens einer damit verbundenen Konnektionsschicht B) miteinander koextrudiert und aus der dabei resultierenden Mehrschichtfolie einen im wesentlichen koaxialen und zylindrischen Mehrschichtschlauch formt.

17. Verfahren nach Anspruch 16, **dadurch gekennzeichnet, daß** man bei der Erzeugung der Mehrschichtfolie noch ein drittes Kunststoffmaterial zur Ausbildung wenigstens einer Deckschicht C) mit hoher Transparenz und geringer Kristallinität und einer Formbeständigkeit von > 121°C koextrudiert, wobei die Deckschicht C) auf einer der beiden Außenseiten ausgebildet wird.

18. Verfahren nach einem der Ansprüche 16 bis 17, **dadurch gekennzeichnet, daß** man den Schlauch nach dem Formen mit Wasser schockkühlt.

19. Verwendung des PVC-freien Mehrschichtschlauches nach einem der Ansprüche 1 bis 15 für medizinische Zwecke.

20. Verwendung nach Anspruch 19 als fluidführende Leitung in der Dialyse, Infusion oder künstlichen Ernährung.

21. Verwendung nach Anspruch 19 als Blutschlauch.

## Claims

1. PVC-free, multi-layer tube for medical purposes having at least two layers of which a base layer A) made of a first plastic material is bonded to at least one connecting layer B), made of a second plastic material, **characterised in that** the first plastic material is preponderantly a synthetic rubber based upon isoprene or polypropylene with a density ρ ≤ 0.9 g / cm³ which is dimensionally stable when subjected to heat sterilisation at ≥ 121 °C, has a Shore Hardness D ≤ 32 and which possesses a residual stress at ≥ 121 °C sufficient to form a press fit at a connection point, such that the base layer A) remains undeformed at temperatures of ≥ 121 °C and that a ring or a loop with a diameter of down to 60 mm can be formed without kinking taking place, while the second plastic material consists predominantly of a polyethylene co-polymer or a synthetic rubber with Mw < 100,000 g/mol which has a tendency to flow under the connection pressure arising during formation of a press fit while undergoing heat sterilisation at 121 °C and possesses a Shore Hardness A of ≤ 65 with the result that each connecting layer B) under connection pressure at a temperature of 121 °C no longer retains its shape.

2. PVC-free multi-layer tube according to claim 1, **characterised by** the sequence of the layers B) A), A) B) or B), A) B) proceeding in each case from the inside to the outside.

3. PVC-free multi-layer tube according to one of the foregoing claims, **characterised in that** the tube exhibits additionally at least one transparent cover layer C) of a third plastic material as the outer layer, where layer C) is arranged either as the innermost or outermost layer.

4. PVC-free multi-layer tube according to claim 3, **characterised in that** the outermost or innermost outer layer is a connecting layer B) and the corresponding opposite outer layer is a cover layer C).

5. PVC-free multi-layer tube according to one of claims 3 or 4 **characterised by** the sequence of the layers B) A) C), C) A) B), C) B) A) B) or B) A) B) C) proceeding in each case from the inside to the outside.

6. PVC-free multi-layer tube according to claim 3 or claim 4, **characterised by** the layers B)A)C) or C)A)B), according to the use of the tube.

7. PVC-free multi-layer tube according to one of the foregoing claims 3 to 6, **characterised in that** the third plastic material contains at least one polymer which is dimensionally stable at temperatures of ≥ 121 °C.

8. PVC-free multi-layer tube according to claim 7, **characterised in that** the third plastic material is dimensionally stable at temperatures of ≥ 121 °C.

9. PVC-free mufti-layer tube according to one of the foregoing claims, **characterised in that** the layers adhere to one another without the use of additional adhesive agents.

10. PVC-free multi-layer tube according to one of the foregoing claims, **characterised in that** the layers are essentially free from plasticisers, anti-blocking agents, anti-static agents and other filler materials.

11. PVC-free multi-layer tube according to one of claims 3 to 10, **characterised in that** the layers A), B) and/or C) each additionally contain up to 40 % by weight relative to 100 % by weight of their content of plastic material, of such plastic material which serves to form one or both of the adjacent layers of the PVC-free multi-layer tube.

12. PVC-free multi-layer tube according to one of the foregoing claims, **characterised in that** the plastic materials for all the layers of the tube are selected such that they consist essentially of polyolefines or of plastic materials based upon olefines.

13. PVC-free multi-layer tube according to one of the foregoing claims, **characterised in that** the base layer A) has a thickness of between 900 and 980 µm.

14. PVC-free multi-layer tube according to one of the foregoing claims, **characterised in that** the connecting layer B) has a thickness of between 10 µm and 50 µm.

15. PVC-free mufti-Dyer tube according to one of claims 4 to 14, **characterised in that** the cover layer C) has a thickness of between 10 µm and 50 µm.

16. Procedure for the manufacture of a PVC-free multi-layer tube for medical purposes according to the claims 1-15 where in order to produce a multi-layer tube containing at least two layers of a multi-layer tube from plastic material, the first plastic material for forming the base layer A) and the second plastic material for forming at least one connecting layer B) bonded to it are co-extruded with one another thus forming from the resulting multi-layer film a multi-layer tube which has an essentially co-axial and cylindrical form.

17. Procedure according to claim 16, **characterised in that** during the production of the multi-layer film, yet a third plastic material is co-extruded to form at least one cover layer C) of a highly transparent nature together with low crystallinity and dimensional stability at > 121 °C, where the coyer layer C) is formed on one of the two outer surfaces.

18. Procedure according to one of the claims 16 to 17, **characterised in that** the tube is shock-cooled with water once its shape has been determined.

19. Use of the PVC-free multi-layer tube according to one of the claims 1 to 15 for medical purposes.

20. Use according to claim 19 as a liquid-conducting pipe in dialysis, infusion or artificial feeding.

21. Use according to claim 19 as a tube for conducting blood.

## Revendications

1. Tuyau flexible multicouche exempt de PVC destiné à des fins médicales comprenant au moins deux couches dont la première est une couche de base A) constituée d'une première matière synthétique à laquelle est reliée au moins une couche de connexion B) constituée d'une deuxième matière synthétique, **caractérisé par le fait que** la première matière synthétique représente, à titre principal, un caoutchouc synthétique à base d'isoprène ou du polypropylène possédant une densité p ≤ 0,9 g/Cc, qui résiste à une stérilisation à la chaleur à une température ≥ 121 °C en l'absence de déformation, qui possède une dureté Shore D ≤ 32 et qui présente, à une température ≥ 121 °C, une tension résiduelle qui suffit pour former un ajustage serré à un endroit de connexion, de telle sorte que la couche de base A), à des températures ≥ 121 °C, manifeste encore une résistance à la déformation et peut être façonnée pour obtenir un cercle ou une boucle possédant un diamètre s'élevant jusqu'à 60 mm en l'absence de dégradation par flambage, tandis que la deuxième matière synthétique représente, à titre principal, un copolymère de polyéthylène ou un caoutchouc synthétique possédant en poids moléculaire Mw < 100.000 g/mole, qui manifeste une tendance à l'écoulement lors d'une stérilisation à la chaleur à une température de 121 °C sous l'influence de la pression de connexion résultant de la mise en oeuvre d'un ajustage serré et présente une dureté Shore A ≤ 65, de telle sorte que chaque couche de connexion, à une température de 121 °C, ne résiste plus à la déformation sous la pression de connexion.

2. Tuyau flexible multicouche exempt de PVC selon la revendication 1, **caractérisé par** la succession de couches B) A), A) B) ou B) A) B), chaque fois de l'intérieur vers l'extérieur.

3. Tuyau flexible multicouche exempt de PVC selon l'une quelconque des revendications précédentes, **caractérisé en ce que** le tuyau flexible présente en outre au moins une couche de recouvrement transparente C) à titre de couche externe constituée par une troisième matière synthétique, la couche C) étant respectivement disposée pour faire office de couche située le plus à l'intérieur ou de couche située le plus à l'extérieur.

4. Tuyau flexible multicouche exempt de PVC selon la revendication 3, **caractérisé en ce que** la couche externe située le plus à l'extérieur ou située le plus à l'intérieur est une couche de connexion B) et la couche externe opposée correspondant à cette dernière est une couche de recouvrement C).

5. Tuyau flexible multicouche exempt de PVC selon la revendication 3 ou 4, **caractérisé par** la succession de couches B) A) C), C) A) B), C) B) A) B) ou B) A) B) C), chaque fois de l'intérieur vers l'extérieur.

6. Tuyau flexible multicouche exempt de PVC selon la revendication 3 ou 4, **caractérisé par** les couches B) A) C) ou C) A) B), en fonction de l'utilisation respective du tuyau flexible.

7. Tuyau flexible multicouche exempt de PVC selon l'une quelconque des revendications précédentes 3 à 6, **caractérisé en ce que** la troisième matière synthétique présente au moins un polymère dont la résistance à la déformation est indiquée à des températures ≥ 121 °C.

8. Tuyau flexible multicouche exempt de PVC selon la revendication 7, **caractérisé en ce que** la troisième matière synthétique présente une résistance à la déformation à des températures ≥ 121 °C.

9. Tuyau flexible multicouche exempt de PVC selon l'une quelconque des revendications précédentes, **caractérisé en ce que** les couches adhèrent l'une à l'autre en l'absence d'un adhésif supplémentaire.

10. Tuyau flexible multicouche exempt de PVC selon l'une quelconque des revendications précédentes, **caractérisé en ce que** les couches sont essentiellement exemptes de plastifiants, d'agents s'opposant à l'adhérence par contact entre feuilles, d'agents antistatiques et d'autres matières de remplissage.

11. Tuyau flexible multicouche exempt de PVC selon l'une quelconque des revendications précédentes 3 à 10, **caractérisé en ce que** les couches A), B) et/ou C) présentent respectivement en outre, jusqu'à concurrence de 40 % en poids rapportés à 100 % en poids de leur matière synthétique, la matière synthétique qui sert à la réalisation d'une couche voisine du tuyau flexible multicouche exempt de PVC ou encore desdites deux couches voisines.

12. Tuyau flexible multicouche exempt de PVC selon l'une quelconque des revendications précédentes, **caractérisé en ce que** les matières synthétiques pour toutes les couches du tuyau flexible sont sélectionnées de telle sorte qu'elles sont constituées essentiellement de polyoléfines ou de matières synthétiques à base de ces dernières.

13. Tuyau flexible multicouche exempt de PVC selon l'une quelconque des revendications précédentes, **caractérisé en ce que** la couche de base A) présente une épaisseur entre 900 et 980 µm.

14. Tuyau flexible multicouche exempt de PVC selon l'une quelconque des revendications précédentes, **caractérisé en ce que** la couche de connexion B) présente une épaisseur entre 10 µm et 50 µm.

15. Tuyau flexible multicouche exempt de PVC selon l'une quelconque des revendications précédentes, **caractérisé en ce que** la couche de recouvrement C) présente une épaisseur entre 10 µm et 50 µm.

16. Procédé pour la fabrication d'un tuyau flexible multicouche exempt de PVC destiné à des fins médicales selon les revendications 1 à 15, dans lequel, pour former un tuyau flexible multicouche en matière synthétique contenant au moins deux couches on soumet à une extrusion conjointe la première matière synthétique pour la formation de la couche de base A) et la deuxième matière synthétique pour la réalisation d'au moins une couche de connexion B) reliée à la première citée et, à partir de la feuille multicouche que l'on obtient en l'occurrence, on forme un tuyau flexible multicouche essentiellement coaxial et cylindrique.

17. Procédé selon la revendication 16, **caractérisé en ce que**, lors de la formation de la feuille multicouche, on soumet encore à une extrusion conjointe, une troisième matière synthétique pour la réalisation d'au moins une couche de recouvrement C) possédant une transparence élevée et une cristallinité minime ainsi qu'une résistance à la déformation > 121 °C, la couche de recouvrement C) étant réalisée sur un des deux côtés externes.

18. Procédé selon l'une quelconque des revendications 16 à 17, **caractérisé en ce qu'**on soumet le tuyau flexible, après sa formation, à un refroidissement ultrarapide avec de l'eau.

19. Utilisation du tuyau flexible multicouche exempt de PVC selon l'une quelconque des revendications 1 à 15 pour des objets médicaux.

20. Utilisation selon la revendication 19, à titre de conduit de passage pour fluide lors d'une dialyse, d'une perfusion ou d'une alimentation artificielle.

21. Utilisation selon la revendication 19, à titre de tuyau flexible pour le sang.
